# EUROPEAN PATENT APPLICATION

(11) **EP 4 424 702 A1**
(43) Date of publication of application: **04.09.2024**
(21) Application number: 21962230.5
(22) Date of filing: 15.12.2021
(51) Int. Cl.: C07K 14/00, G01N 33/68, C12N 15/11

(54) **WHOLE GENETIC CODING NAD+ PROTEIN PROBE BASED ON RESONANCE ENERGY TRANSFER, AND PREPARATION METHOD THEREFOR AND USE THEREOF**

(30) Priority: 29.10.2021 CN 202111273571
(71) Applicant: Shenzhen Nadical Technology Co., Ltd, Shenzhen, Guangdong 518107 (CN)
(72) Inventor: CHEN, Liuqing, Shenzhen, Guangdong 518055 (CN); YU, Qiuliyang, Shenzhen, Guangdong 518055 (CN)
(74) Representative: Cabinet Chaillot
(86) International application number: PCT/CN2021/138560
(87) International publication number: WO 2023/070887

(57) **Abstract**

A fully genetically encoded NAD+protein probe based on resonance energy transfer, and a preparation method therefor and the use thereof. Specifically provided is a fully genetically encoded NAD+protein probe, which is formed by means of linking a resonance energy transfer donor, an NAD+responsive protein and a resonance energy transfer receptor in series, wherein the NAD+ responsive protein is a mutant of a DNA ligase; the sequence of the mutant of the DNA ligase is as shown in SEQ ID NO.3 or SEQ ID NO.6; the resonance energy transfer donor is selected from luciferase or a fluorescent protein; the resonance energy transfer receptor is selected from a fluorescent protein; and the fluorescent protein as the resonance energy transfer receptor is different from that of the resonance energy transfer donor. The protein probe can be synthesized in living cells and used for detecting the concentration of NAD+ in living cells.

## Description

### Technical Field

The invention relates to the technical field of biological probe, in particular to a fully genetically encoded NAD+ protein probe based on resonance energy transfer, and a preparation method therefor and the use thereof.

### Background Art

At present, the NAD + content in biological samples can be detected as follows:

### 1) NAD + colorimetric assay kit:

The NAD+ colorimetric assay kit firstly utilizes enzymatic reaction to convert NAD+ into NADH completely, then utilizing the generated NADH to reduce a dye molecule to change its absorbance, and determining the concentration of dye molecule by spectrophotometer to infer the concentration of NAD+ in the sample.

### 2) high performance liquid chromatography-mass spectrometry:

In the high performance liquid chromatography-mass spectrometry method, different components in a sample to be detected are separated by using the high performance liquid chromatography method, and absorbance of different components are detected at the same time. Meanwhile, different components enter the mass spectrum through ionization at the time of high performance liquid chromatography, different components are qualitatively compared by using nucleoplasm, and different components are quantitatively determined by using ion strength.

### 3) fluorescent protein probes based on changes in fluorescence intensity:

Protein engineering is utilized to design a fluorescent protein probe for sensing NAD+. The method generally fuses NAD+ sensor protein and fluorescent protein, and utilizes conformational change generated after specific binding of the NAD+ sensor protein and NAD+ to regulate fluorescence protein conformation, which further affects optical characteristics of fluorescent protein such as quantum yield, thereby enabling the strength of the fluorescence signal emitted by the protein changes along with the change of the concentration of NAD+ in the system.

### 4) Semi-synthetic NAD+ protein probes based on resonance energy transfer:

The method utilizes the fluorescence or bioluminescence resonance energy transfer principle to detect NAD+ concentration, and uses recombinant protein (protein portion) and synthetic fluorescent ligand molecule (synthetic portion) to construct semi-synthetic protein probe for NAD+ detection. Such kind of probe is formed by fusing luciferase (or fluorescent protein), NAD+ binding protein and self-labeling protein, and the self-labeling protein is linked to a synthetic molecule via covalent bond, wherein the synthetic molecule contains a red fluorogen and a ligand molecule for the NAD+ binding protein. Therefore, in the natural state, the NAD+ binding protein in the sensor protein does not bind to the ligand molecule, the probe is in the open state, the fluorogen and the luciferase (or fluorescent protein) are distant, and the energy resonance transfer does not occur, and the probe as a whole emits the original emission light of luciferase (or fluorescent protein). When the concentration of NAD+ in the system increases, the NAD+ binding protein binds to the ligand molecule, bringing the fluorogen and the probe as a whole luciferase (or fluorescent protein) closer, and the energy resonance transfer occurs, so that the whole probe emits red light of the fluorogen. The color of the light emitted by the probe changes with the concentration of NAD+ in the sample, therefore, the ratio of light intensity of the luciferase (or fluorescent protein) and red fluorogen can be measured to quantify the NAD+ concentration in the system.

NAD+ colorimetric assay kits and high performance liquid chromatography-mass spectrometry methods are unable to measure NAD+ concentrations in living cells. A single assay based on these methods requires the lysis of a large number of cells (about 1 million), which loses temporal and spatial information of cellular NAD+ concentration; the sample lysis process alters the intracellular NAD+ concentration, resulting in inaccurate results; in addition, the detection of a single sample takes 10 to 30 minutes and relies on large-scale instrument, which severely limits the detection of a large number of samples and is a rate-limiting step in the study of NAD+ metabolism and in medicinal development.

Fluorescent probe-based methods for measuring NAD+ in living cells have not been able to measure NAD+ dynamic change in living cells and subcellular structures for extended periods of time, primarily because current fluorescent probes rely on fluorescent microscopic image reading, and the phototoxicity for exciting fluorescence renders it difficult to achieve long-time measurements of living cells. At the same time, the use of these probes depends on large precision instruments such as fluorescence microscopes, which limits the detection of large quantities of samples. In addition, such probes use fluorescence intensity signal with single wavelength to quantify the NAD+ concentration, which leads to the dynamic differences in the expression of the probe molecules, causing obvious interference in the NAD+ quantification. Therefore, these probes rely on internal standard fluorescent protein of other colors as a calibration tool, and the using process thereof is relatively complicated.

### Summary of the Invention

### Technical Problem

Semi-synthetic NAD+ protein probe based on resonance energy transfer utilize the principle of resonance energy transfer to quantify NAD+ concentration and realize self-calibrating, no longer relying on single wavelength, but rather on a two-wavelength ratio of light intensity to quantify the concentration of the substance to be measured. However, since this type of probe consists of a recombinant protein and a synthetic fluorescent ligand molecule, the cell can only autonomously express the fusion protein part of the probe when carrying out NAD+ detection in living cells, while the synthetic molecule needs to be supplied extracellularly. The dependence on extracellular synthesized molecules limits the application of this type of probe in conditions such as prolonged observation of a large number of living cell samples.

### Technical Solution to the Problem

### Technical Solution

In terms of probe function, the invention 1) achieves fully genetically encoded of the probe structure, accomplishing resonance energy transfer NAD+ quantification independent of synthesizing fluorescent ligand molecule, and 2) achieves self-calibration of the probe signal, i.e., NAD+ quantification based on the ratio of two-wavelength light intensity.

The invention provides several fully genetically encoded NAD+ molecular probes based on resonance energy transfer. The series of molecular probes are capable of responding to NAD+ molecules in vitro and intracellularly and have the advantages of high specificity, suitable C50 value (the concentration of substance to be measured at the time that results in a 50% change in the conformation of the probe) and high dynamic range.

In one hand, the invention provides a fully genetically encoded NAD+ protein probe based on resonance energy transfer, which is formed by means of linking a resonance energy transfer donor, an NAD+ responsive protein and a resonance energy transfer receptor in series, wherein the NAD+ responsive protein is a mutant of a DNA ligase; the sequence of the mutant of the DNA ligase is as shown in SEQ ID NO.3 or SEQ ID NO.6; the resonance energy transfer donor is selected from luciferase or a fluorescent protein; the resonance energy transfer receptor is selected from a fluorescent protein; and the fluorescent protein as the resonance energy transfer receptor is different from that of the resonance energy transfer donor.

Further, the resonance energy transfer donor is selected from the circularly permuted bioluminescent protein cpNLuc (circularly permuted Nano Luciferase) or the green fluorescent protein mNeoGreen. Further, the circularly permuted bioluminescent protein cpNLuc is the bioluminescent protein cpNLuc that introducing G4V mutation. More preferably, the mutant sequence of cpNLuc is SEQ ID NO.4 or SEQ ID NO.8. The sequence of green fluorescent protein mNeoGreen is SEQ ID NO.10 or SEQ ID NO.13.

Further, the resonance energy transfer receptor is selected from the green fluorescent protein mNeoGreen or the red fluorescent protein mScarlet. Still further, the red fluorescent protein mScarlet is the red fluorescent protein mScarlet with residue G225 deleted therein . More preferably, the mutant sequence of mScarlet is SEQ ID NO. 2 or SEQ ID NO. 12. The sequence of the green fluorescent protein mNeoGreen is SEQ ID NO. 10 or SEQ ID NO. 13.

Further, said NAD+ protein probe is formed by means of linking an mScarlet mutant, a LigA mutant and a cpNLuc mutant in series, preferably, the amino acid sequence thereof is SEQ ID NO.1 , SEQ ID NO.5 or SEQ ID NO.7.

Further, said NAD+ protein probe is formed by means of linking an mNeoGreen mutant, a LigA mutant and a cpNLuc mutant in series, preferably, the amino acid sequence thereof is SEQ ID NO.9.

Further, said NAD+ protein probe is composed of an mScarlet mutant, a LigA mutant and an mNeoGreen mutant protein, preferably, the amino acid sequence thereof is SEQ ID NO.11.

In terms of probe structure, the invention 1) designs a protein domain whose conformation is strongly regulated by NAD+, i.e., NAD+ responsive protein, and 2) fuses the N-terminal and C-terminal ends of the NAD+ responsive protein to the donor and receptor capable of forming resonance energy transfer phenomenon, respectively, The resonance energy transfer donor and receptor are a luciferase and a fluorescent protein, respectively, or two fluorescent proteins with different colors. 3) The linkage between the NAD+ responsive protein and the resonance energy transfer donor and receptor is optimized to maximize the dynamic range of the probe. 4) The binding site of the NAD+ responsive protein and NAD+ is optimized to modulate the concentration range of the probe for NAD+ detection.

Another aspect of the invention is to provide the use of the above-described NAD+ protein probe in the preparation of reagent for the detection of NAD+ concentration.

A further aspect of the invention is to provide a composition for detecting concentration of NAD+, said composition comprising said NAD+ molecular probe.

Further, said composition comprises a cushion fluid.

Further, said composition comprises a bioluminescent substrate, such as Furimazine.

A further aspect of the invention provides a nucleotide sequence for encoding said NAD+ protein probe.

A further aspect of the invention provides a vector, said vector including said nucleotide sequence.

Preferably, said vector is a lentiviral expression vector, such as pCDH-CMV-MCS-EF1-Neo vector or a pc DNA3.1 vector.

A further aspect of the invention provides a cell capable of expressing said NAD+ protein probe.

Preferably, said cell is obtained after transfection of said vector into the living cell and said vector is capable of translating and acquiring said NAD+ protein probe within the cell.

Preferably, said living cell may be selected from any human or animal cell, for example selected from HEK293, CHO, and Hep G2, etc.

A further aspect of the invention provides the use of said cell in the preparation of experimental model for studying NAD+.

Preferably, said experimental model is used to study NAD+ agonists or inhibitors.

A further aspect of present invention provides a method for detecting NAD+ concentration, said method comprising the following steps:
S11) employing said protein probe in the invention mixed with the reagent for detecting NAD+ concentration,
S12) detecting the light intensity at the maximum luminescence wavelength of the resonance energy transfer donor and the resonance energy transfer receptor in the probe, respectively, and calculate the ratio of light intensity of the two;
S13) regressing on a standard curve to obtain the corresponding NAD+ concentration; or
S13) detecting the ratio of light intensities at different time points and obtaining the trend of changes in NAD+ concentration at different time points; or
S13) detecting the change in the ratio of light intensity after addition of different active ingredients, and obtaining the effect of different active ingredients on changes in NAD+ concentration.

Preferably, in step S11), when the resonance energy transfer donor in said protein probe is a luciferase, a bioluminescent substrate also needs to be added before detecting the light intensity.

Preferably, the standard curve in step S13) is prepared by using NAD+ with different standard concentrations, detecting the light intensity ratios corresponding to the different concentrations of NAD+ by steps S11-S12 respectively, then the logarithmic value of the NAD+ concentration is used as the horizontal coordinate, and the light intensity ratio is used as the vertical coordinate to make a standard curve.

A further aspect of the invention provides a method for detecting the concentration of NAD+ in a living cell, said method comprising the following steps:
S21) transfecting said vector into the cell to be tested by slow-virus infection method, and screen and obtain the stably transfected cell line to be tested through the fluorescent signal of fluorescent protein as a marker;
S22) detecting the light intensity at the maximum luminescence wavelength of the resonance energy transfer donor and the resonance energy transfer receptor in the nucleotide-encoded NAD+ protein probe in the vector, respectively, and calculate the ratio of light intensity of the two;
S23) regressing on a standard curve to obtain the corresponding intracellular NAD+ concentration; or
S23) detecting the ratio of light intensities at different time points and obtaining the trend of changes in NAD+ concentration at different time points; or
S23) detecting the change in the ratio of light intensity after addition of different active ingredients and obtaining the effect of different active ingredients on changes of intracellular NAD+ concentration.

In terms of probe application, the invention constructs a cell line stably expressing the NAD+ resonance energy transfer protein probe to achieve representation of intracellular NAD+ concentration in living cells.

### Advantages Effects of the Invention

### Advantages Effects

The invention innovatively realizes a fully genetically encoded NAD+ protein probe based on resonance energy transfer. In the background art, the fully genetically encoded NAD+ protein probe is not based on the principle of resonance energy transfer, therefore merely relying on the light intensity emitted by fluorescent protein to realize NAD+ quantification, making it difficult to rule out the obvious interference on the quantification of NAD+ caused by dynamic difference in expression quantity of the probe molecule. Another background art uses a semi-synthetic design to achieve NAD+ quantification based on resonance energy transfer, which solves the interference caused by expression quantity of the probe molecule, but the dependence on non-genetic coding synthetic molecule limits the application of semi-synthetic probes in living cells. The challenge with fully encoded probe, as opposed to semi-synthetic probe (wherein a portion of the probe is not amino acid sequence and cannot be encoded in the cell), is the difficulty in finding suitable protein fragments and designing methods to achieve large conformational changes comparable to those of semi-synthetic probes. However, the invention achieves this breakthrough. Moreover, the NAD+ protein probe provided by the invention not only realize NAD+ quantification based on resonance energy transfer and fully genetically encoded of the probe, i.e., self-calibration of the probe signals, i.e., NAD+ quantification based on the ratio of light intensity of two wavelengths, but also realize NAD+ monitoring in living cells independent of synthesized fluorescent ligand molecules.

At the same time, the invention specifies a new amino acid sequence of NAD+ protein probe with better functionality. Compared to the background art, the fully genetically encoded NAD+ protein probe provided by the invention possesses a completely different amino acid sequence. Wherein, the NAD+ responsive protein used in this probe is a novel DNA ligase LigA mutant containing structural domain interface residue mutation (R68N/R163D or R68N/R163P). Meanwhile, this probe uses a novel linkage approach to link NAD+ responsive protein with resonance energy transfer donor and receptor, specifically manifests as: deletion of residue G225 of mScarlet, residue P5 of LigA, and residues G1, L2, and S3 of cpNluc, and introduction of the G4V mutation in the cpNluc component.

### Brief Description of Accompany Drawings

### Description of Accompany Drawings

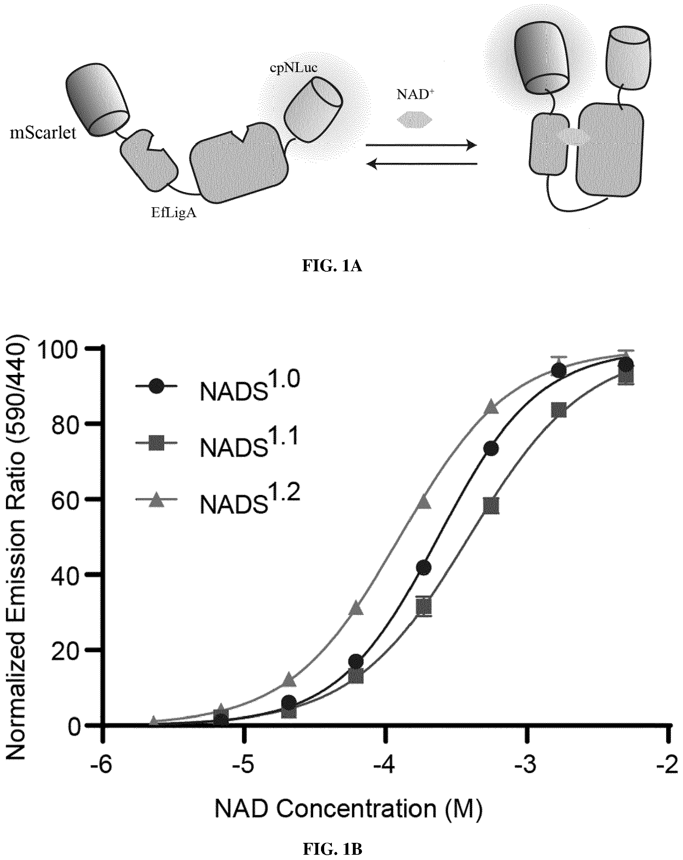

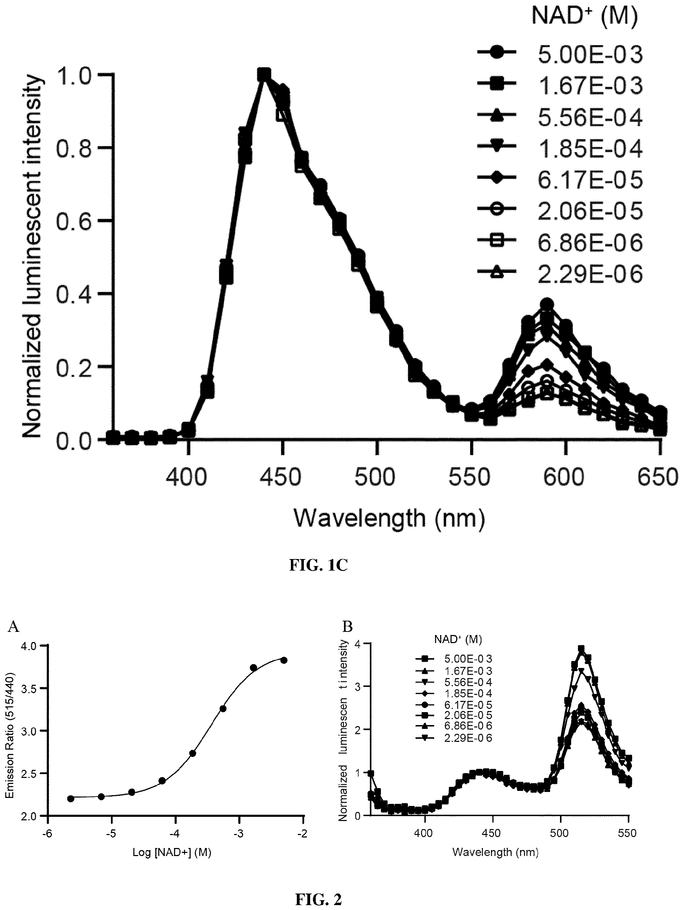

FIG. 1A is the schematic diagram of NAD+ molecular probe detecting the concentration of NAD+ molecules based on resonance energy transfer provided by the invention. In the invention, as shown in FIG. 1A, when the NAD+ responsive protein does not bind the NAD+ molecule, the probe structure is in an "open" state, which results in a longer distance between the resonance energy transfer donor and the receptor, with lower resonance energy transfer efficiency, and the whole probe emits light from the resonance energy transfer donor. When NAD+ responsive protein binds NAD+ molecules, its conformation changes from an "open" to a "closed" state, which leads to the resonance energy transfer donor and the receptor being close to each other, resulting in a higher resonance energy transfer efficiency, and the probe emits the light from the resonance energy transfer receptor. The change in the resonance energy transfer efficiency caused by the NAD+ molecule is ultimately manifested as a change in the wavelengths intensity emitted by the resonance energy transfer donor and receptor in the probe. This emitted light intensity ratio further indicates the concentration of NAD+ molecules in the system. FIG. 1B shows the change in the peak ratio at 590 nm and 440 nm of the purified NAD+ molecular probes NADS1.0, NADS 1.1, and NADS1.2 in response to different concentrations of NAD+. The horizontal coordinate is the logarithmic value of NAD+ concentration and the vertical coordinate is the ratio of light intensity at normalized 590 nm and 440 nm. FIG. 1C is the bioluminescent spectrogram of purified NAD+ molecular probes NADS1.0, NADS1.1, and NADS 1.2 responding to different concentrations of NAD+ (represented by NADS 1.2), with wavelengths (nm) in the horizontal coordinate and normalized bioluminescent intensity in the vertical coordinate.
FIG. 2(A) shows the change in the peak ratio at 515 nm and 440 nm of the purified NAD+ molecular probe NADS2.0 in response to different concentrations of NAD+. The horizontal coordinate is the logarithmic value of NAD+ concentration and the vertical coordinate is the ratio of light intensity at 515 nm and 440 nm. FIG. 2 (B) is the bioluminescent spectrogram of purified NAD+ molecular probe NADS2.0 in response to different concentrations of NAD+ with wavelength (nm) in horizontal coordinate and normalized bioluminescent intensity in the vertical coordinate.

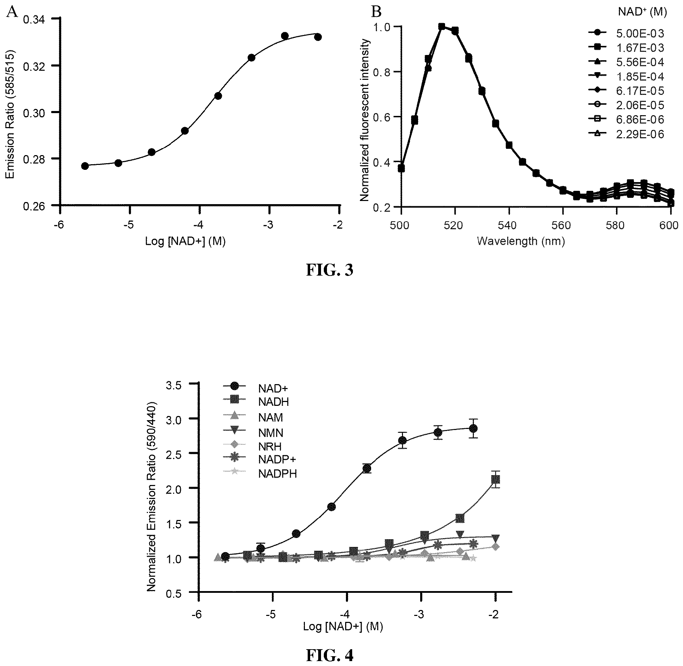

FIG. 3(A) shows the change in the peak ratio at 585 nm and 515 nm of the purified NAD+ molecular probe NADS3.0 in response to different concentrations of NAD+. The horizontal coordinate is the logarithmic value of NAD+ concentration and the vertical coordinate is the ratio of light intensity at 585 nm and 515 nm. (B) is the bioluminescent spectrogram of purified NAD+ molecular probe NADS3.0 in response to different concentrations of NAD+ with wavelength (nm) in horizontal coordinate and normalized bioluminescent intensity in the vertical coordinate.
FIG. 4 shows the change in peak ratio at 590 nm and 440 nm of the purified NAD+ molecular probe NADS1.2 in response to different NAD+ analogs. The horizontal coordinate is the logarithmic value of NAD+ concentration and the vertical coordinate is the the ratio of light intensity at normalized 590 nm and 440 nm.

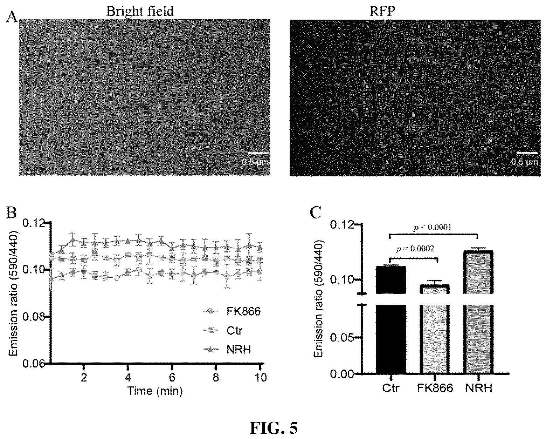

FIG. 5(A) is a bright field and RFP fluorescent image of probe NADS1.0 transfected into HEK293 cells. FIG. 5(B) is the diagram illustrating light intensity ratios at 590 nm and 440 nm changing with time for the stably-transfected HEK293 control group and different compound-treated groups. FIG. 5(C) is the diagram of the light intensity ratios at average 590 nm and 440 nm for the stably-transfected HEK293 control group and different compound-treated groups, and all P-values are calculated using unpaired two-tailed Student's t tests.

### Embodiment of the Invention

### Implementation of the Invention

In order to make the aforementioned objects, features and advantages of the invention more comprehensible, specific embodiments of the invention are described in detail hereinafter, but it should not be construed as a limitation to the implementable range thereof.

### Embodiment 1 preparation of NAD + molecular probe

Preparing an NAD + molecular probe, and the probe sequence is shown as follows:
The sequence of NADS ^{1.0} is shown in SEQ ID NO.1

mScarlet mutant-LigA mutant-cpNluc mutant.

MVSKGEAVIKEFMRFKVHMEGSMNGHEFEIEGEGEGRPYEGTQTAKLKVTKG
GPLPFSWDILSPQFMYGSRAFTKHPADIPDYYKQSFPEGFKWERVMNFEDGGAVTVT
QDTSLEDGTLIYKVKLRGTNFPPDGPVMQKKTMGWEASTERLYPEDGVLKGDIKMA
LRLKDGGRYLADFKTTYKAKKPVQMPGAYNVDRKLDITSHNEDYTVVEQYERSEG
RHSTLTLTAATTRAQELRKQLNQYSHEYYVKDQPSVEDYVYDRLYKELVDIETEFPD
LITPDSPTQNVGGKVLSGFEKAPHDIPMYSLNDGFSKEDIFAFDERVRKAIGKPVAYC
CELLIDGLAISLRYENGVFVRGATRGDGTVGENITENLRTVRSVPMDLTEPISVEVRG
ECYMPKQSFVALNEEREENGQDIFANPRNAAAGSLRQLDTKIVAKRNLNTFLYTVA
DFGPMKAKTQFEALEELSAIGFRTNPERQLCQSIDEVWAYIEEYHEKRSTLPYEINGIV
IKVNEFALQDELGFTVKAPRWAIAYKFPGDQMGQIEKIFKVVYPVDDHHFKVILHYG
TLVIDGVTPNMIDYFGRPYEGIAVFDGKKITVTGTLWNGNKIIDERLINPDGSLLFRVT
INGVTGWRLCERILAGGTGGSGGTGGSMVFTLEDFVGDWRQTAGYNLDQVLEQGG
VSSLFQNLGVSVTPIQRIVLSGENGLKIDIHVIIPYE SEQ ID NO.1

Wherein, the sequence of mScarlet mutant 1 is shown as SEQ ID NO.2:

MVSKGEAVIKEFMRFKVHMEGSMNGHEFEIEGEGEGRPYEGTQTAKLKVTKGGPLP
FSWDILSPQFMYGSRAFTKHPADIPDYYKQSFPEGFKWERVMNFEDGGAVTVTQDT
SLEDGTLIYKVKLRGTNFPPDGPVMQKKTMGWEASTERLYPEDGVLKGDIKMALRL
KDGGRYLADFKTTYKAKKPVQMPGAYNVDRKLDITSHNEDYTVVEQYERSEGRHS
T SEQ ID NO.2

The sequence of LigA mutant 1 is shown as SEQ ID NO.3:

LTLTAATTRAQELRKQLNQYSHEYYVKDQPSVEDYVYDRLYKELVDIETEFP
DLITPDSPTQNVGGKVLSGFEKAPHDIPMYSLNDGFSKEDIFAFDERVRKAIGKPVAY
CCELLIDGLAISLRYENGVFVRGATRGDGTVGENITENLRTVRSVPMDLTEPISVEVR
GECYMPKQSFVALNEEREENGQDIFANPRNAAAGSLRQLDTKIVAKRNLNTFLYTV

ADFGPMKAKTQFEALEELSAIGFRTNPERQLCQSIDEVWAYIEEYHEKRSTLPYEINGI
VIKVNEFALQDELGFTVKAPRWAIAYKFP SEQ ID NO.3

The sequence of cpNluc mutant 1 is shown as SEQ ID NO.4:

GDQMGQIEKIFKVVYPVDDHHFKVILHYGTLVIDGVTPNMIDYFGRPYEGIAVF
DGKKITVTGTLWNGNKIIDERLINPDGSLLFRVTINGVTGWRLCERILAGGTGGSGGT
GGSMVFTLEDFVGDWRQTAGYNLDQVLEQGGVSSLFQNLGVSVTPIQRIVLSGENG
LKIDIHVIIPYE SEQ ID NO.4

The sequence of NADS ^{1.1} is shown as SEQ ID NO.5

mScarlet mutant-LigA mutant-cpNluc mutant.

MVSKGEAVIKEFMRFKVHMEGSMNGHEFEIEGEGEGRPYEGTQTAKLKVTKG
GPLPFSWDILSPQFMYGSRAFTKHPADIPDYYKQSFPEGFKWERVMNFEDGGAVTVT
QDTSLEDGTLIYKVKLRGTNFPPDGPVMQKKTMGWEASTERLYPEDGVLKGDIKMA
LRLKDGGRYLADFKTTYKAKKPVQMPGAYNVDRKLDITSHNEDYTVVEQYERSEG
RHSTLTLTAATTRAQELRKQLNQYSHEYYVKDQPSVEDYVYDRLYKELVDIETEFPD
LITPDSPTQNVGGKVLSGFEKAPHDIPMYSLNDGFSKEDIFAFDERVRKAIGKPVAYC
CELLIDGLAISLRYENGVFVRGATRGDGTVGENITENLRTVRSVPMPLTEPISVEVRG
ECYMPKQSFVALNEEREENGQDIFANPRNAAAGSLRQLDTKIVAKRNLNTFLYTVA
DFGPMKAKTQFEALEELSAIGFRTNPERQLCQSIDEVWAYIEEYHEKRSTLPYEINGIV
IKVNEFALQDELGFTVKAPRWAIAYKFPGDQMGQIEKIFKVVYPVDDHHFKVILHYG
TLVIDGVTPNMIDYFGRPYEGIAVFDGKKITVTGTLWNGNKIIDERLINPDGSLLFRVT
INGVTGWRLCERILAGGTGGSGGTGGSMVFTLEDFVGDWRQTAGYNLDQVLEQGG
VSSLFQNLGVSVTPIQRIVLSGENGLKIDIHVIIPYE SEQ ID NO.5

The sequence of mScarlet mutant 1 is shown as SEQ ID NO.2: 
MVSKGEAVIKEFMRFKVHMEGSMNGHEFEIEGEGEGRPYEGTQTAKLKVTKG
GPLPFSWDILSPQFMYGSRAFTKHPADIPDYYKQSFPEGFKWERVMNFEDGGAVTVT
QDTSLEDGTLIYKVKLRGTNFPPDGPVMQKKTMGWEASTERLYPEDGVLKGDIKMA
LRLKDGGRYLADFKTTYKAKKPVQMPGAYNVDRKLDITSHNEDYTVVEQYERSEG
 RHST SEQ ID NO.2

The sequence of LigA mutant 2 is shown as SEQ ID NO.6:

LNQYSHEYYVKDQPSVEDYVYDRLYKELVDIETEFPDLITPDSPTQNVGGKVL
SGFEKAPHDIPMYSLNDGFSKEDIFAFDERVRKAIGKPVAYCCELLIDGLAISLRYEN
GVFVRGATRGDGTVGENITENLRTVRSVPMPLTEPISVEVRGECYMPKQSFVALNEE
REENGQDIFANPRNAAAGSLRQLDTKIVAKRNLNTFLYTVADFGPMKAKTQFEALE
ELSAIGFRTNPERQLCQSIDEVWAYIEEYHEKRSTLPYEINGIVIKVNEFALQDELGFT
VKAPRWAIAYKFP SEQ ID NO.6

The sequence of cpNluc mutant 1 is shown as SEQ ID NO.4:

GDQMGQIEKIFKVVYPVDDHHFKVILHYGTLVIDGVTPNMIDYFGRPYEGIAV
FDGKKITVTGTLWNGNKIIDERLINPDGSLLFRVTINGVTGWRLCERILAGGTGGSGG
TGGSMVFTLEDFVGDWRQTAGYNLDQVLEQGGVSSLFQNLGVSVTPIQRIVLSGEN
GLKIDIHVIIPYE SEQ ID NO.4

The sequence of NADS ^{1.2} is shown as SEQ ID NO.7

mScarlet mutant-LigA mutant-cpNluc mutant

MVSKGEAVIKEFMRFKVHMEGSMNGHEFEIEGEGEGRPYEGTQTAKLKVTKG
GPLPFSWDILSPQFMYGSRAFTKHPADIPDYYKQSFPEGFKWERVMNFEDGGAVTVT
QDTSLEDGTLIYKVKLRGTNFPPDGPVMQKKTMGWEASTERLYPEDGVLKGDIKMA
LRLKDGGRYLADFKTTYKAKKPVQMPGAYNVDRKLDITSHNEDYTVVEQYERSEG
RHSTLTLTAATTRAQELRKQLNQYSHEYYVKDQPSVEDYVYDRLYKELVDIETEFPDL
ITPDSPTQNVGGKVLSGFEKAPHDIPMYSLNDGFSKEDIFAFDERVRKAIGKPVAYCCE
LLIDGLAISLRYENGVFVRGATRGDGTVGENITENLRTVRSVPMDLTEPISVEVRGEC
YMPKQSFVALNEEREENGQDIFANPRNAAAGSLRQLDTKIVAKRNLNTFLYTVADFG
PMKAKTQFEALEELSAIGFRTNPERQLCQSIDEVWAYIEEYHEKRSTLPYEINGIVIKV
NEFALQDELGFTVKAPRWAIAYKFPVDQMGQIEKIFKVVYPVDDHHFKVILHYGTLVI
DGVTPNMIDYFGRPYEGIAVFDGKKITVTGTLWNGNKIIDERLINPDGSLLFRVTINGV
TGWRLCERILAGGTGGSGGTGGSMVFTLEDFVGDWRQTAGYNLDQVLEQGGVSSL

FQNLGVSVTPIQRIVLSGENGLKIDIHVIIPYE SEQ ID NO.7

The sequence of mScarlet mutant 1 is shown as SEQ ID NO.2:

MVSKGEAVIKEFMRFKVHMEGSMNGHEFEIEGEGEGRPYEGTQTAKLKVTKG
GPLPFSWDILSPQFMYGSRAFTKHPADIPDYYKQSFPEGFKWERVMNFEDGGAVTVT
QDTSLEDGTLIYKVKLRGTNFPPDGPVMQKKTMGWEASTERLYPEDGVLKGDIKMA
LRLKDGGRYLADFKTTYKAKKPVQMPGAYNVDRKLDITSHNEDYTVVEQYERSEG
RHST SEQ ID NO.2

The sequence of LigA mutant 1 is shown as SEQ ID NO.3:

LTLTAATTRAQELRKQLNQYSHEYYVKDQPSVEDYVYDRLYKELVDIETEFP
DLITPDSPTQNVGGKVLSGFEKAPHDIPMYSLNDGFSKEDIFAFDERVRKAIGKPVAY
CCELLIDGLAISLRYENGVFVRGATRGDGTVGENITENLRTVRSVPMDLTEPISVEVR
GECYMPKQSFVALNEEREENGQDIFANPRNAAAGSLRQLDTKIVAKRNLNTFLYTV
ADFGPMKAKTQFEALEELSAIGFRTNPERQLCQSIDEVWAYIEEYHEKRSTLPYEINGI
VIKVNEFALQDELGFTVKAPRWAIAYKFP SEQ ID NO.3

The sequence of cpNluc mutant 2 is shown as SEQ ID NO.8:

VDQMGQIEKIFKVVYPVDDHHFKVILHYGTLVIDGVTPNMIDYFGRPYEGIAV
FDGKKITVTGTLWNGNKIIDERLINPDGSLLFRVTINGVTGWRLCERILAGGTGGSGG
TGGSMVFTLEDFVGDWRQTAGYNLDQVLEQGGVSSLFQNLGVSVTPIQRIVLSGEN
GLKIDIHVIIPYE SEQ ID NO.8

The sequence of NADS ^{2.0} is shown as SEQ ID NO.9

mNeoGreen mutant-LigA mutant-cpNluc mutant

MASLPATHELHIFGSINGVDFDMVGQGTGNPNDGYEELNLKSTKGDLQFSPWI
LVPHIGYGFHQYLPYPDGMSPFQAAMVDGSGYQVHRTMQFEDGASLTVNYRYTYE
GSHIKGEAQVKGTGFPADGPVMTNSLTAADWCRSKKTYPNDKTIISTFKWSYTTGN
GKRYRSTARTTYTFAKPMAANYLKNQPMYVFRKTELKHSKTELNFKEWQKAFTDK
LTLTAATTRAQELRKQLNQYSHEYYVKDQPSVEDYVYDRLYKELVDIETEFPDLITP
DSPTQNVGGKVLSGFEKAPHDIPMYSLNDGFSKEDIFAFDERVRKAIGKPVAYCCEL

LIDGLAISLRYENGVFVRGATRGDGTVGENITENLRTVRSVPMDLTEPISVEVRGECY
MPKQSFVALNEEREENGQDIFANPRNAAAGSLRQLDTKIVAKRNLNTFLYTVADFGP
MKAKTQFEALEELSAIGFRTNPERQLCQSIDEVWAYIEEYHEKRSTLPYEINGIVIKVN
EFALQDELGFTVKAPRWAIAYKFPVDQMGQIEKIFKVVYPVDDHHFKVILHYGTLVI
DGVTPNMIDYFGRPYEGIAVFDGKKITVTGTLWNGNKIIDERLINPDGSLLFRVTING
VTGWRLCERILAGGTGGSGGTGGSMVFTLEDFVGDWRQTAGYNLDQVLEQGGVSS
LFQNLGVSVTPIQRIVLSGENGLKIDIHVIIPYE SEQ ID NO.9

The sequence of mNeoGreen1 mutant is shown as SEQ ID NO.10:

MASLPATHELHIFGSINGVDFDMVGQGTGNPNDGYEELNLKSTKGDLQFSPWI
LVPHIGYGFHQYLPYPDGMSPFQAAMVDGSGYQVHRTMQFEDGASLTVNYRYTYE
GSHIKGEAQVKGTGFPADGPVMTNSLTAADWCRSKKTYPNDKTIISTFKWSYTTGN
GKRYRSTARTTYTFAKPMAANYLKNQPMYVFRKTELKHSKTELNFKEWQKAFTDK
SEQ ID NO.10

The sequence of LigA1 mutant is shown as SEQ ID NO.3:

LTLTAATTRAQELRKQLNQYSHEYYVKDQPSVEDYVYDRLYKELVDIETEFP
DLITPDSPTQNVGGKVLSGFEKAPHDIPMYSLNDGFSKEDIFAFDERVRKAIGKPVAY
CCELLIDGLAISLRYENGVFVRGATRGDGTVGENITENLRTVRSVPMDLTEPISVEVR
GECYMPKQSFVALNEEREENGQDIFANPRNAAAGSLRQLDTKIVAKRNLNTFLYTV
ADFGPMKAKTQFEALEELSAIGFRTNPERQLCQSIDEVWAYIEEYHEKRSTLPYEINGI
VIKVNEFALQDELGFTVKAPRWAIAYKFP SEQ ID NO.3

] The sequence of cpNluc mutant 2 is shown as SEQ ID NO.8:

VDQMGQIEKIFKVVYPVDDHHFKVILHYGTLVIDGVTPNMIDYFGRPYEGIAV
FDGKKITVTGTLWNGNKIIDERLINPDGSLLFRVTINGVTGWRLCERILAGGTGGSGG
TGGSMVFTLEDFVGDWRQTAGYNLDQVLEQGGVSSLFQNLGVSVTPIQRIVLSGEN
GLKIDIHVIIPYE SEQ ID NO.8

The sequence of NADS3.0 is shown as SEQ ID NO.11

mScarlet mutant-LigA mutant-mNeoGreen mutant

MVSKGEAVIKEFMRFKVHMEGSMNGHEFEIEGEGEGRPYEGTQTAKLKVTKG
GPLPFSWDILSPQFMYGSRAFTKHPADIPDYYKQSFPEGFKWERVMNFEDGGAVTVT
QDTSLEDGTLIYKVKLRGTNFPPDGPVMQKKTMGWEASTERLYPEDGVLKGDIKMA
LRLKDGGRYLADFKTTYKAKKPVQMPGAYNVDRKLDITSHNEDYTVVEQYERSEG
RHLTLTLTAATTRAQELRKQLNQYSHEYYVKDQPSVEDYVYDRLYKELVDIETEFP
DLITPDSPTQNVGGKVLSGFEKAPHDIPMYSLNDGFSKEDIFAFDERVRKAIGKPVAY
CCELLIDGLAISLRYENGVFVRGATRGDGTVGENITENLRTVRSVPMDLTEPISVEVR
GECYMPKQSFVALNEEREENGQDIFANPRNAAAGSLRQLDTKIVAKRNLNTFLYTV
ADFGPMKAKTQFEALEELSAIGFRTNPERQLCQSIDEVWAYIEEYHEKRSTLPYEINGI
VIKVNEFALQDELGFTVKAPRWAIAYKFPPPATHELHIFGSINGVDFDMVGQGTGNP
NDGYEELNLKSTKGDLQFSPWILVPHIGYGFHQYLPYPDGMSPFQAAMVDGSGYQV
HRTMQFEDGASLTVNYRYTYEGSHIKGEAQVKGTGFPADGPVMTNSLTAADWCRS
KKTYPNDKTIISTFKWSYTTGNGKRYRSTARTTYTFAKPMAANYLKNQPMYVFRKT
ELKHSKTELNFKEWQKAFTDVMGMDELYK SEQ ID NO.11

The sequence 2 of mScarlet is shown as SEQ ID NO.12:

MVSKGEAVIKEFMRFKVHMEGSMNGHEFEIEGEGEGRPYEGTQTAKLKVTKG
GPLPFSWDILSPQFMYGSRAFTKHPADIPDYYKQSFPEGFKWERVMNFEDGGAVTVT
QDTSLEDGTLIYKVKLRGTNFPPDGPVMQKKTMGWEASTERLYPEDGVLKGDIKMA
LRLKDGGRYLADFKTTYKAKKPVQMPGAYNVDRKLDITSHNEDYTVVEQYERSEG
RHLT SEQ ID NO.12

The sequence of LigA mutant 1is shown as SEQ ID NO.3:

LTLTAATTRAQELRKQLNQYSHEYYVKDQPSVEDYVYDRLYKELVDIETEFP
DLITPDSPTQNVGGKVLSGFEKAPHDIPMYSLNDGFSKEDIFAFDERVRKAIGKPVAY
CCELLIDGLAISLRYENGVFVRGATRGDGTVGENITENLRTVRSVPMDLTEPISVEVR
GECYMPKQSFVALNEEREENGQDIFANPRNAAAGSLRQLDTKIVAKRNLNTFLYTV
ADFGPMKAKTQFEALEELSAIGFRTNPERQLCQSIDEVWAYIEEYHEKRSTLPYEINGI
VIKVNEFALQDELGFTVKAPRWAIAYKFP SEQ ID NO.3

The sequence of mNeoGreen mutant 2 is shown as SEQ ID NO.13:

PPATHELHIFGSINGVDFDMVGQGTGNPNDGYEELNLKSTKGDLQFSPWILVP
HIGYGFHQYLPYPDGMSPFQAAMVDGSGYQVHRTMQFEDGASLTVNYRYTYEGSH
IKGEAQVKGTGFPADGPVMTNSLTAADWCRSKKTYPNDKTIISTFKWSYTTGNGKR
YRSTARTTYTFAKPMAANYLKNQPMYVFRKTELKHSKTELNFKEWQKAFTDVMG
MDELYK SEQ ID NO.13

Embodiment 2 a fully genetically encoded NAD+ molecular probe based on bioluminescent resonance energy transfer (BRET) used for measurement of NAD+ content in sample.

To verify that the NAD+ molecular probes NADS1.0, NADS1.1, NADS1.2, and NADS2.0 provided by the invention are capable of being used for the measurement of NAD+ content in sample, titration experiments are performed as shown in the following steps:
(1) Prepare the following sample
   Probe solution: the purified NAD+ molecular probe is diluted with HEPES cushion fluid (50 mM NaCl, 50 mM HEPES, pH 7.2) to 4 nM, and set aside temporarily in an ice box;
   NAD+ solution: NAD+ solutions of different concentrations (50 mM, 16.7 mM, 5.56 mM, 1.85 mM, 617 µM, 206 µM, 68.6 µM and 22.9 µM) are prepared using a 3-fold continuous gradient dilution method, the cushion fluid used is HEPES cushion fluid, stored in an ice box;
   Bioluminescent substrate solution: dilute the bioluminescent substrate solution 100-fold with water and store in an ice box, keeping in dark place.
(2) Add 80 µL of probe solution, 10 µL of NAD+ solution, and 10 µL of bioluminescent substrate solution into the white 96-well elisa plate, blowing, suction, and mix immediately several times (at least 8 times) with multi-channel pipette; use Flex Station3 multifunctional microplate reader to read the light intensity values at the wavelengths of 440 nm and 590 nm (NADS1.0, NADS1.1, and NADS1.2), or 515 nm (NADS2.0) under the luminescence mode, and monitor the values for 5 min continuously, and the average receptor-to-donor luminescence ratio is calculated for this time period. The luminescent spectrum of the probe at different NAD+ concentrations are obtained by monitoring the light intensity values in the wavelength range between 360 and 650 nm.

As can be seen in FIG. 1B, the 590/440 light intensity ratios of the probes NADS1.0, NADS1.1, and NADS1.2 are different under different NAD+ concentrations, which increases along with increase of concentration within certain NAD+ concentrations (10 µM-1 mM). FIG. 1C shows the results of scanning spectrum at different wavelengths, and it can be seen that there are two obvious absorption peaks at 590 nm and 440 nm for the donor and the receptor, respectively. The results of FIG. 1 also show that the probes of the invention are NAD+ molecule that can quantitatively measure physiological concentration range, and by introducing different mutations into the LigA mutant, the invention designs and produces probe versions with different affinities, NADS ^{1.0}, NADS ^{1.1} and NADS ^{1.2}, whose C₅₀ values (the concentration of the substance to be measured that results in a 50% conformation change of the probe) are 235 µM , 388 µM , and 126 µM , which can be used for NAD+ detection scenarios in different concentration ranges.

The results for NADS2.0 are shown by FIG. 2, from which it can be seen that the 515/440 light intensity values of probe NADS2.0 are different under different NAD+ concentrations and become larger with increasing concentration over a certain NAD+ concentration range (10 µM-1 mM). FIG. 2B shows the results of scanning spectrum at different wavelengths, and it can be seen that there are two obvious absorption peaks at 515 nm and 440 nm. Similar to the results of the Embodiment, probe of this embodiment also has a high dynamic range (1.8-fold) , with C₅₀ value at 364 µM.

It is illustrated that the NAD+ molecular probes NADS1.0, NADS1.1, NADS1.2, and NADS2.0 of the invention have different light intensity ratios at different wavelengths, and can be used to obtain the NAD+ concentration by regression calculations with different light intensity ratios. It can be used for the measurement of NAD+ content, and the concentration range can be relatively wide.

Embodiment 3 a fully genetically encoded NAD+ molecular probe based on bioluminescent resonance energy transfer (BRET) used for measurement of NAD+ content in sample.

To verify that the NAD+ molecular probes NADS3.0 provided by the invention are capable of being used for the measurement of NAD+ content in sample, titration experiments are performed as shown in the following steps:
(1) Prepare the following sample
   Probe solution: the purified NAD+ molecular probe is diluted with HEPES cushion fluid (50 mM NaCl, 50 mM HEPES, pH 7.2) to 2 nM, and set aside temporarily in an ice box;
   NAD+ solution: NAD+ solutions of different concentrations (50 mM, 16.7 mM, 5.56 mM, 1.85 mM, 617 µM, 206 µM, 68.6 µM and 22.9 µM) are prepared using a 3-fold continuous gradient dilution method, the cushion fluid used is HEPES cushion fluid, stored in an ice box;
(2) Add 90 µL of probe solution and 10 µL of NAD+ solution into the black 96-well elisa plate, blowing, suction, and mix immediately several times (at least 8 times) with multi-channel pipette; the excitation wavelength is set to 470 nm and the emission wavelengths at 515 nm and 585 nm are measured with Flex Station3 multifunctional microplate reader under the luminescence mode, and monitor the values for 5 min continuously, and the average luminescence ratio of 585 nm to 515 nm is calculated for this time period. The luminescent spectrum of the probe at different NAD+ concentrations are obtained by monitoring the light intensity values in the wavelength range between 500 and 600 nm.

As can be seen in FIG. 3, the 585/515 light intensity ratios of the probe NADS3.0 are different under different NAD+ concentrations, which increases along with increase of concentration within certain NAD+ concentrations (10 µM-1 mM). It is shown that the NAD+ molecular probe NADS3.0 of the invention can be used for the measurement of NAD+ content. Probe NADS3.0 is a NAD+ molecular probe based on fluorescence resonance energy transfer with a dynamic range of 1.21-fold and C₅₀ value of 162 µM.

Embodiment 4 Response of NAD+ molecular probe to different NAD+ analogs.

To verify that the NAD+ molecular probe provided by the invention are highly selective, NAD1.2 is taken as an example and titration experiments are performed as shown in the following steps:
(1) Prepare the following sample
   Probe solution: the purified NAD+ molecular probe is diluted with HEPES cushion fluid (50 mM NaCl, 50 mM HEPES, pH 7.2) to 4 nM, and set aside temporarily in an ice box;
   NAD+ and its analogs solution: NAD+, NADP+, and NADPH solutions of different concentrations (50 mM, 16.7 mM, 5.56 mM, 1.85 mM, 617 µM, 206 µM, 68.6 µM and 22.9 µM), NADH, NMN and NRH solutions of different concentrations (40 mM,13.3 mM, 4.4 mM,1.4 mM, 494 µM, 165 µM, 54.9µM and 18.3 µM) are prepared using a 3-fold continuous gradient dilution method, the cushion fluid used is HEPES cushion fluid, stored in an ice box;
   Bioluminescent substrate solution: dilute the bioluminescent substrate solution 100-fold with water and store in an ice box, keeping in dark place.
(2) Add 80 µL of probe solution, 10 µL of NAD+ solution or 10 µL of NAD+ analogs solution, and 10 µL of bioluminescent substrate solution into the white 96-well elisa plate, blowing, suction, and mix immediately several times (at least 8 times) with multi-channel pipette; use Flex Station3 multifunctional microplate reader to read the light intensity values at the wavelengths of 440 nm and 590 nm under the luminescence mode, and monitor the values for 5 min continuously, and the average receptor-to-donor luminescence ratio is calculated for this time period.

The experimental result is shown in FIG. 4, the experimental results show that the probe of the invention is highly selective for NAD+ molecules. The probe did not respond to NAM and NADPH; the probe responded to NADH and NRH, but C₅₀ values thereof is high(>1 mM); and the probe responded to NADP+ and NMN molecules with a small dynamic range.

Embodiment 5 Mammalian cell lines stably expressing NAD+ molecular probes used to measure dynamic changes in NAD+ concentration in living cells.

The NAD+ molecular probe provided by the invention can detect changes in the concentration of NAD+ molecules in cells. The coding gene of this probe (NADS1.0) is cloned into the pCDH-CMV-MCS-EF1-Neo vector, and the HEK293 stably transfected cell line is prepared using lentiviral method. HEK293 cells are spread in 96-well white cell culture plates at an inoculum size of 10,000 cells per well with 100 µL of DMEM medium (high sugar, phenol red free), and cultures for 24h at 37□ with 5% CO₂, and then the stably transfected cell lines are treated with FK866 (a NAMPT inhibitor, which can effectively reduce the level of intracellular NAD+) and NRH (precursor of NAD+, which can effectively increase the level of intracellular NAD+), at a final concentration of 10 nM and 50 µM, respectively. After 24h of treatment with the compounds, the DMEM medium (high sugar, phenol red free) is changed to fresh DMEM medium (high sugar, phenol red free, bioluminescent substrate diluted by 1,000-fold) with bioluminescent substrate. The emitted light intensity at 590 nm and 440 nm is measured using Flex Station3 multifunctional microplate reader. According to the detection principle of the probe, the ratio of emitted light intensity at 590 nm and 440 nm indicates the NAD+ concentration in living cells. It is found in the detection that the ratio decreased in cells treated with FK866, indicating a decrease in intracellular NAD+ concentration, while in the NRH-treated cells, the ratio increased, indicating an increase in intracellular NAD+ concentration. This change is as expected, indicating that the NAD+ probe is able to respond to changes of intracellular NAD+ concentration (FIG. 5).

## Claims

1. A fully genetically encoded NAD+ protein probe based on resonance energy transfer, which is formed by means of linking a resonance energy transfer donor, an NAD+ responsive protein and a resonance energy transfer receptor in series;
wherein the NAD+ responsive protein is a mutant of a DNA ligase; the sequence of the mutant of the DNA ligase is as shown in SEQ ID NO.3 or SEQ ID NO.6;
the resonance energy transfer donor is selected from luciferase or a fluorescent protein; the resonance energy transfer receptor is selected from a fluorescent protein; and the fluorescent protein as the resonance energy transfer receptor is different from that of the resonance energy transfer donor.

2. The fully genetically encoded NAD+ protein probe of claim 1 wherein the resonance energy transfer donor is selected from the circularly permuted bioluminescent protein cpNLuc or the green fluorescent protein mNeoGreen;
preferably, the circularly permuted bioluminescent protein cpNLuc is the bioluminescent protein cpNLuc that introducing G4V mutation;
more preferably, the mutant sequence of cpNLuc is SEQ ID NO.4 or SEQ ID NO.8. the sequence of green fluorescent protein mNeoGreen is SEQ ID NO.10 or SEQ ID NO.13.

3. The fully genetically encoded NAD+ protein probe of claim 1 wherein the resonance energy transfer receptor is selected from the green fluorescent protein mNeoGreen or the red fluorescent protein mScarlet;
preferably, the red fluorescent protein mScarlet is the red fluorescent protein mScarlet with residue G225 deleted therein;
more preferably, the mutant sequence of mScarlet is SEQ ID NO.2 or SEQ ID NO.12; the sequence of the green fluorescent protein mNeoGreen is SEQ ID NO.10 or SEQ ID NO.13.

4. The fully genetically encoded NAD+ protein probe of claim 1 wherein said NAD+ protein probe is formed by means of linking an mScarlet mutant, a LigA mutant and a cpNLuc mutant in series; or
said NAD+ protein probe is formed by means of linking an mNeoGreen mutant, a LigA mutant and a cpNLuc mutant in series; or
said NAD+ protein probe is composed of an mScarlet mutant, a LigA mutant and an mNeoGreen mutant protein;
preferably, the amino acid sequence thereof is SEQ ID NO.1, SEQ ID NO.5, SEQ ID NO.7, SEQ ID NO.9, or SEQ ID NO.11.

5. A use of the NAD+ protein probe as described in any claim from claim 1 to 4 in the preparation of reagent for the detection of NAD+ concentration.

6. A composition for detecting concentration of NAD+ wherein said composition comprises the NAD+ protein probe as described in any claim from claim 1 to 4;
preferably, said composition also comprises a bioluminescent substrate.

7. A nucleotide sequence for encoding the NAD+ protein probe as described in any claim from claim 1 to 4.

8. A vector, wherein said vector includes the nucleotide sequence of claim 7;
preferably, said vector is a lentiviral expression vector.

9. A cell capable of expressing the NAD+ protein probe as described in any claim from claim 1 to 4;
preferably, said cell is obtained after transfection of the vector as said in claim 8 into the living cell, and said vector is capable of translating and acquiring the NAD+ protein probe as described in any claim from claim 1 to 4 within the cell.

10. The use of the cell as said in claim 9 in the preparation of experimental model;
preferably, said experimental model is used to study agonists or inhibitors in the process of NAD+ synthesis and metabolism.

11. A method for detecting NAD+ concentration, wherein said method comprising the following steps:
S11) employing said protein probe in the invention mixed with the reagent for detecting NAD+ concentration as described in any claim from claim 1 to 4;
S12) detecting the light intensity at the maximum luminescence wavelength of the resonance energy transfer donor and the resonance energy transfer receptor in the probe, respectively, and calculate the ratio of light intensity of the two;
S13) regressing on a standard curve to obtain the corresponding NAD+ concentration; or
S13) detecting the ratio of light intensities at different time points and obtaining the trend of changes in NAD+ concentration at different time points; or
S13) detecting the change in the ratio of light intensity after addition of different active ingredients, and obtaining the effect of different active ingredients on changes in NAD+ concentration;
preferably, in step S11), when the resonance energy transfer donor in said protein probe is a luciferase, a bioluminescent substrate also needs to be added before detecting the light intensity;
preferably, the standard curve in step S13) is prepared by using NAD+ with different standard concentrations, detecting the light intensity ratios corresponding to the different concentrations of NAD+ by steps S11-S12 respectively, then the logarithmic value of the NAD+ concentration is used as the horizontal coordinate, and the light intensity ratio is used as the vertical coordinate to make a standard curve.

12. A method for detecting the concentration of NAD+ in a living cell, said method comprising the following steps:
S21) transfecting said vector as said in claim 8 into the cell to be tested by slow-virus infection method, and screen and obtain the stably transfected cell line to be tested through the fluorescent signal of fluorescent protein as a marker;
S22) detecting the light intensity at the maximum luminescence wavelength of the resonance energy transfer donor and the resonance energy transfer receptor in the nucleotide-encoded NAD+ protein probe in the vector, respectively, and calculate the ratio of light intensity of the two;
S23) regressing on a standard curve to obtain the corresponding intracellular NAD+ concentration; or
S23) detecting the ratio of light intensities at different time points and obtaining the trend of changes in NAD+ concentration at different time points; or
S23) detecting the change in the ratio of light intensity after addition of different active ingredients and obtaining the effect of different active ingredients on changes of intracellular NAD+ concentration.
